# EUROPEAN PATENT APPLICATION

(11) **EP 4 302 754 A1**
(43) Date of publication of application: **10.01.2024**
(21) Application number: 22762626.4
(22) Date of filing: 04.03.2022
(51) Int. Cl.: A61K 9/14, A61K 9/19, A61K 9/16, A61K 31/337, A61P 35/00

(54) **STABLE DOCETAXEL ALBUMIN NANOPARTICLE COMPOSITION**

(30) Priority: 05.03.2021 CN 202110243298
(71) Applicant: CSPC Zhongqi Pharmaceutical Technology (Shijiazhuang) Co., Ltd., Shijiazhuang, Hebei 050035 (CN)
(72) Inventor: LI, Chunlei, Shijiazhuang, Hebei 050035 (CN); ZHANG, Xiaojun, Shijiazhuang, Hebei 050035 (CN); ZHAO, Yuanyuan, Shijiazhuang, Hebei 050035 (CN); CHEN, Dongjian, Shijiazhuang, Hebei 050035 (CN); LIANG, Min, Shijiazhuang, Hebei 050035 (CN); XING, Qianbin, Shijiazhuang, Hebei 050035 (CN); LI, Nan, Shijiazhuang, Hebei 050035 (CN); SHU, Yun, Shijiazhuang, Hebei 050035 (CN)
(74) Representative: Gille Hrabal Partnerschaftsgesellschaft mbB Patentanwälte
(86) International application number: PCT/CN2022/079286
(87) International publication number: WO 2022/184164

(57) **Abstract**

A composition containing a docetaxel albumin nanoparticle. The composition contains docetaxel and an acid-denatured albumin, wherein the acid-denatured albumin is obtained by means of adding an acid to human serum albumin to adjust the pH value. The composition can be prepared into an injection or a freeze-dried powder injection. A composition with satisfactory physical and chemical stability is obtained by means of controlling the content of sodium octanoate in human serum albumin, preferably in an acid denaturation condition.

## Description

### TECHNICAL FIELD

The present disclosure belongs to the field of pharmaceutical formulations, and particularly relates to a composition comprising a docetaxel albumin nanoparticle.

### BACKGROUND

Docetaxel is produced by semi-synthesizing a non-cytotoxic precursor (10-deacetyl baccatin III) extracted from needles of *Taxus chinensis,* which is a paclitaxel analog. It acts on cell microtubules to be capable of promoting the tubulin polymerization, inhibiting microtubule depolymerization at the same time, arresting cells in the G2/M phase, and inhibiting mitosis, thereby killing tumor cells. Docetaxel has stronger anti-tumor activity than paclitaxel.

Docetaxel has relatively poor water solubility, and the current commercially available formulation is a common injection thereof, which was first developed by Sanofi with the trade name of TAXOTERE^{®}. Docetaxel is conventionally administered by intravenous infusion over 1 hour at a dose of 75 mg/m² every 3 weeks. However, the docetaxel injection, represented by TAXOTERE^{®}, has the following disadvantages due to the use of ethanol and Tween-80:
(1)A severe hypersensitivity reaction may happen; thus, the dexamethasone premedication is required, resulting in poor patient compliance. Tween-80 can cause severe hypersensitivity reactions and fluid retention and administration of an anti-allergic drug in advance is required. For example, 16 mg of dexamethasone is administered every day for at least 3 days from one day before drip infusion to prevent the allergic reaction and fluid retention. Even if dexamethasone is administered in advance, there are still reports of the severe allergic reaction. It is explicitly mentioned in the specification that "severe fluid retention has been reported in 6.5% (6/92) of the patients who received 3 days of dexamethasone prophylactic treatment".
(2) Ethanol may affect the central nervous system, and slowing the infusion rate is required to alleviate symptoms of intoxication. In 2014, the FDA published "Drug Safety Communication on the Intravenous Chemotherapy Docetaxel Containing Ethanol and Intoxication Risk", thought that ethanol in docetaxel injection can affect the central nervous system and slowing the infusion rate may alleviate symptoms of ethanol intoxication, and called for development of ethanol-free docetaxel formulations.
(3) Tween-80 causes an allergic reaction and can only be administered at a low concentration. The patient compliance is poor. Tween-80 can cause an allergic reaction, and can cause a hemolytic reaction when the concentration is too high, so Tween-80 can be diluted to a relatively low concentration for administration, the infusion time is prolonged, and the patient compliance is poor.
(4) The product has poor compatibility stability, and an infusion device without PVC material is required, which is inconvenient for clinical use. It is mentioned in the specification that "docetaxel pre-injection (10 mg/mL) should be used immediately after preparation. However, its physicochemical properties show that the pre-injection can be stable for 8 h whether it is stored at 2-8 °C or at room temperature. The injection (no more than 0.74 mg/mL) should be used within 4 h at room temperature and administered by intravenous drip for 1 h", which is inconvenient for clinical use; it is explicitly mentioned in the specification that the use of PVC infusion devices is not recommended and the contact with PVC infusion devices can lead to the leaching of bis-(2-ethylhexyl)phthalate in the PVC infusion devices, presenting a potential safety hazard.

To avoid the many disadvantages of the common formulations, Nanoxel-PM^{™} (marketed in Korea, trade name: Nanoxel^{®} M), developed by Samyang company in Korea, was disclosed in the document "Development of docetaxel-loaded intravenous formulation, Nanoxel-PM™ using polymer-based delivery system"( Sa-Won Lee, Min-Hyuk Yun, Seung Wei Jeong, Journal of Controlled Release, 155 (2011) 262-271). This product uses the polymer, polyethylene glycol-poly(D,L-lactide) (mPEG-PDLLA), as the carrier to prepare docetaxel micelles with an average particle size of 10-50 nm and uniform particle size distribution. The clinic use mode of the product is disclosed on the official website. In order to minimize the allergic reaction, a pre-treatment is required, for example, 16 mg dexamethasone is administered orally every day for 3 days consecutively from one day before administration (https://www.samyangbiopharm.com/eng/ProductIntroduce/injection03). Although this product is ethanol-free and Tween 80-free, the clinical premedication is still required.

Human serum albumin (abbreviated as HSA) is a protein in the human plasma, consists of 585 amino acids, and has a molecular weight of 66 kDa. The concentration of human serum albumin in plasma is 42 g/L, and accounts for about 60% of total plasma proteins. In body fluids, the human serum albumin can transport fatty acids, bile pigments, amino acids, steroid hormones, metal ions, and many therapeutic molecules, etc. Meanwhile, the human serum albumin can maintain the normal osmotic pressure and physiological pH value in blood.

Proteins are macromolecules composed of various amino acids,wherein amino acids link orderly through peptide bonds and disulfide bond, which is referred to as the primary structure; hydrogen bonds can be formed between amino groups and acyl groups inter or intra polypeptide chains, so that the main polypeptide chain has a certain regular conformation, including α-helix, β-sheet, β-turn, Ω-loop, and the like, which are referred to as the secondary structures of the proteins; the peptide chain is further coiled and folded on the basis of the secondary structures to form a complete spatial conformation which is referred to as the tertiary structure; the spatial structure formed by the aggregation of multiple peptide chains through non-covalent bonds is referred to as the quaternary structure, in which one peptide chain is called a subunit.

Protein denaturation is the effect of alterations in the native structure and properties by physical or chemical disruptions. It is generally believed that the secondary structure and the tertiary structure of the protein are altered or destroyed as a result of the denaturation. Protein denaturation methods are mainly divided into chemical methods and physical methods, wherein the chemical methods include adding strong acids, strong bases, heavy metal salts, urea, acetone, etc.; the physical methods include heating, ultraviolet and X-ray irradiation, ultrasonication, vigorous oscillation or stirring, etc. Michael Dockal, Daniel C. Carter, and Florian Rüker reported in the paper "Conformational Transitions of the Three Recombinant Domains of Human Serum Albumin Depending on pH" (J Biol Chem, 2000, 275(5): 3042-3050) that the human serum albumin can undergo conformational transitions at different pH (such as N-F and F-E transitions under acidic conditions and N-B transition under alkaline conditions), leading to the denaturation of the protein. In the present disclosure, the albumin denatured by adding acids is defined as "acid-denatured albumin" and the pH of the albumin solution is less than 5.5.

The production process of the human serum albumin, whether imported or domestic, is substantially the same. The human serum albumin is prepared by purifying healthy human plasma via a low-temperature ethanol protein separation method and heating at 60 °C for 10 h to inactivate viruses, sodium caprylate is usually added to the commercially available albumin as a thermal protective agent, for example, 0.16 mmol of sodium caprylate is added to 1 g of albumin.

Since the human serum albumin is an endogenous substance in the human body and has good biocompatibility, it can be used as a natural carrier of hydrophobic drugs and can increase the solubility of water-insoluble drugs. Paclitaxel for injection (albumin-bound, trade name: Abraxane^{®}), developed by Abraxis BioScience in the United States with the human serum albumin as an auxiliary material via an emulsification method, has been approved by the FDA in 2005 for the treatment of breast cancer after failure of combination chemotherapy for metastatic disease or relapse within 6 months of adjuvant chemotherapy, and subsequently approved for the treatment of non-small cell lung cancer, pancreatic cancer, and gastric cancer (Japan).

Compared with the paclitaxel injection (Taxol^{®}), the formulation of Abraxane^{®} does not comprise polyoxyethylene castor oil which can cause severe hypersensitivity reactions, so it is not required to premedicate anti-allergic drugs. Moreover, Abraxane^{®} can be quickly administered at high concentrations, shortening the infusion time to 30 min, and significantly improving the patient compliance: due to the improvement of safety, the dosage of paclitaxel can be increased from 175 mg/m² to 260-300 mg/m²; the polyoxyethylene castor oil in Taxol^{®} may inhibit the binding of paclitaxel to albumin, and the formulation without polyoxyethylene castor oil can take full advantage of the unique "gp60-caveolin-SPARC" pathway of the albumin to increase delivery of drugs to tumors, thereby increasing the efficacy. At present, researchers in various countries are trying to develop human serum albumin-based nanoparticles carrying other drugs, such as docetaxe.

The patent US2005/0004002A1, published by Neil P. Desai et al., discloses a docetaxel albumin nanoparticle prepared by an emulsification method, the formulation of which only comprises docetaxel and a human serum albumin, the particle size is 50-220 nm which is substantially similar before and after the lyophilization. However, the suspension before and after lyophilization are only stable within a short time and cannot meet the clinical requirements.

Adding soybean oil, phospholipid, cholesterol, benzoic acid, and the like as stabilizers to the formulation to improve the suspension stability was first tried in CN1 03054798A, but all failed. After excessive trials, it is finally confirmed that the stability of the suspension can be significantly improved by adding sodium citrate or a composition of sodium citrate and sodium chloride. However, the large amount of salts leads to high osmotic pressure and strong irritation of the reconstituted suspension, causing obvious pain during injection, and even leading to osmotic damage to local tissue cells after injection, which is inconvenient for clinical use.

The invention also indicates that the nanoparticle suspensions prepared from the anhydrous docetaxel are more stable than those prepared from the docetaxel with bound-water (such as docetaxel trihydrate or docetaxel hemihydrate). Example 28 described that in the absence of stabilizers, when the active ingredient was anhydrous docetaxel (i.e., docetaxel), the nanoparticle suspensions precipitated on the first day of storage at 4 °C, and the sedimentation of the hemihydrate and trihydrate occurred in a shorter time; in the presence of stabilizers, when the active ingredient was anhydrous docetaxel (i.e., docetaxel), the nanoparticle suspensions did not precipitate at 4 °C for up to 2 days, and the sedimentation of the hemihydrate and trihydrate occurred in a shorter time. Therefore, the patent limits the preparation of albumin nanoparticles by the anhydrous docetaxel, which greatly limits the selection range of starting docetaxel form.

The pH of formulations was also investigated in the patent, and it was found that "the increase of pH over 6 increased the physical stability measured in terms of nanoparticle size and sedimentation of the formulation while at the same time increased the amount of degradation of docetaxel to 7-epidocetaxel at room temperature. The acceptable pH range for the physical stability and chemical stability was between 6 and 8.5, the favorable pH range was 6.5-8, and the optimal pH range was 7.25-7.75."

Although CN103054798A believes that the addition of sodium citrate or a combination of sodium citrate and sodium chloride is beneficial to the physical stability of docetaxel albumin nanoparticle formulations, the Chinese Patent CN106137969A indicates that the addition of tartaric acid, citric acid, ascorbic acid, and other organic acids with a pKa of 2.5-4.5, and the like to the docetaxel albumin compositions cannot effectively inhibit the increase of 7-epidocetaxel, and even the increase of 7-epidocetaxel occurs, resulting in poor chemical stability of the compositions and affecting the safety of the formulations. However, the selective addition of substances like amino acids enables the docetaxel albumin to be stable for a long time, significantly inhibiting the production of 7-epidocetaxel. In particular, when the amino acid is arginine, the content of 7-epidocetaxel is only 0.72% after 30 months. The reconstituted suspension of the prepared product can be stable for more than 8 h at room temperature. The patent defines that in the docetaxel albumin nanoparticle pharmaceutical compositions, the amino acid and docetaxel are in a mass ratio of no less than 0.5, preferably no less than 1.

WO2018/059304A1 also discloses a method for improving the product quality of an albumin pharmaceutical composition, wherein the pharmaceutical composition comprises albumin and at least a kind of amino acid with a relative molecular weight of 145-175 or a salt thereof. The amino acid can be one or combination of arginine, histidine, and lysine, preferably arginine and/or histidine, and more preferably arginine. The amino acid or the salt thereof and the albumin are in a weight ratio of 0.1:1-10:1. The amino acid or the salt thereof inhibits the production or increase of an albumin dimer in the preparation, storage, and use of the pharmaceutical composition. The albumin pharmaceutical composition can effectively reduce undesirable responses in human bodies caused by albumin aggregates and dimers in clinical use, such as rash, urticaria, an allergic reaction and possible immune responses, and further ensure the clinical medication safety.

In summary, the improvement of the product quality of the docetaxel albumin requires considering both physical stability (suspension stability) and chemical stability (the degradation of docetaxel and the increase of albumin aggregates). In the docetaxel albumin compositions disclosed in the prior art, one is that a large amount of chelating agent is added as a stabilizer to improve the physical stability of the product, but the effect on the chemical stability of the product is not investigated; in addition, a large amount of chelating agent improves the stability and forms a hypertonic solution at the same time, which is inconvenient for clinical use. The other is that a large amount of amino acid is required to be added into the product, so that the degradation of docetaxel and the increase of albumin aggregates can be inhibited, but the effect on the physical stability is not deeply researched, and only that the reconstituted suspension can be stable for more than 8 h at room temperature is mentioned.

Therefore, it is urgent in the art to find a method that can simultaneously ensure the physical stability and chemical stability of the docetaxel albumin composition.

### SUMMARY

The present disclosure provides a composition comprising a docetaxel albumin nanoparticle, which comprises docetaxel and an acid-denatured albumin.

Alternatively, the present disclosure provides a composition comprising a docetaxel albumin nanoparticle, which is prepared from docetaxel and an acid-denatured albumin.

Alternatively, the present disclosure provides a composition comprising a docetaxel albumin nanoparticle, which is prepared from docetaxel and an acid-denatured albumin, and optionally comprises a tonicity adjusting agent or a pH adjuster,
wherein the docetaxel is preferably anhydrous docetaxel, docetaxel hemihydrate, or docetaxel trihydrate.

The acid-denatured albumin is obtained by adding an acid to human serum albumin to adjust to an appropriate pH value and then denaturing, and preferably:
(1) the acid is selected from an acidic amino acid or acidic polypeptide, an organic acid, and an inorganic acid. The acidic amino acid or acidic polypeptide includes, but is not limited to cysteine hydrochloride, glutathione, etc.; the organic acid includes, but is not limited to citric acid, tartaric acid, etc.; the inorganic acid includes, but is not limited to hydrochloric acid, sulfuric acid, etc. The acid is preferably cysteine hydrochloride, glutathione, or hydrochloric acid, and more preferably cysteine hydrochloride; and/or
(2) the appropriate pH value is preferably 3.5-5.5, preferably 3.5-5.0, more preferably 3.8-4.7, and more preferably 4.0-4.5; and/or
(3) the docetaxel (on an anhydrous basis) and the human serum albumin are in a mass ratio of 1:(2.0-10.0), preferably 1 :(3.0-7.0), and more preferably 1:(4.0-6.0); and/or
(4) the content of sodium caprylate in the human serum albumin is no more than 0.08 mmol/g protein, preferably 0.03-0.08 mmol/g protein, further preferably 0.04-0.08 mmol/g protein, and much more preferably 0.04-0.07 mmol/g protein.

In some embodiments, the docetaxel albumin nanoparticle has a particle size of about 60-200 nm, preferably 90-150 nm, and more preferably 90-135 nm.

The composition comprising the docetaxel albumin nanoparticle described herein optionally comprises a tonicity adjusting agent, so as to regulate the osmotic pressure within an appropriate range. The type of the tonicity adjusting agent is not particularly limited, for example, it can be selected from sodium chloride, glucose, phosphate, and citrate, etc., preferably sodium chloride. The type and amount of the tonicity adjusting agent can be determined by those skilled in the art according to the type and amount of the diluent or the reconstitution medium used clinically. In some embodiments, the tonicity adjusting agent is sodium chloride, and the sodium chloride and the docetaxel are in a weight ratio of (0.75-9):1, preferably (1-7):1, preferably (1.5-4.5):1, and most preferably 2.25:1.

Optionally, the composition described herein comprises a pH adjuster, so as to adjust the pH value within an appropriate range, such that a suspension from which the composition described herein is prepared and/or a reconstituted suspension of the composition of the present disclosure is stable at 25 °C for at least 24 h, preferably at least 30 h, and/or stable at 2-8 °C for at least 7 days, preferably at least 10 days. The "stable" as used herein means that there is no sedimentation of the nanoparticles or turbidity of the suspensions. The type of the pH adjuster is not particularly limited. Preferably, the pH value range is 3.4-5.8, preferably 3.6-5.6, or 3.8-5.0, and more preferably 3.9-4.8.

In some embodiments, the composition described herein is a suspension, wherein the suspension has a pH value of 3.4-5.8, preferably 3.6-5.6, or 3.8-5.0, and more preferably 3.9-4.8. The docetaxel albumin nanoparticle in the suspension has a particle size of about 60-200 nm, preferably 90-150 nm, and more preferably 90-135 nm. The suspension comprises 0-1.8% (w/v), preferably 0.45%-1.8% (w/v), and more preferably 0.9%-1.8% (w/v), of sodium chloride. In some embodiments, the suspension comprises 2-10 mg/mL, preferably 2-8 mg/mL of docetaxel. The suspension is stable at 25 °C for at least 24 h, preferably at least 30 h, and stable at 2-8 °C for at least 7 days, preferably at least 10 days.

In other embodiments, the composition described herein is a lyophilized powder. The docetaxel albumin nanoparticle has a particle size of about 60-200 nm, preferably 90-150 nm, and more preferably 90-135 nm. In some embodiments, the lyophilized powder comprises sodium chloride, and the sodium chloride and the docetaxel are in a weight ratio of (0.75-9):1, preferably (1-7):1, preferably (1.5-4.5):1, and most preferably 2.25:1.

The lyophilized powder is obtained by lyophilizing a suspension comprising the docetaxel albumin nanoparticle, wherein the suspension has a pH value of 3.4-5.8, preferably 3.6-5.6, or 3.8-5.0, and more preferably 3.9-4.8. The docetaxel albumin nanoparticle in the suspension has a particle size of about 60-200 nm, preferably 90-150 nm, and more preferably 90-135 nm. The suspension comprises 0-1.8% (w/v), preferably 0.45%-1.8% (w/v), and more preferably 0.9%-1.8% (w/v), of sodium chloride. The suspension is stable at 25 °C for at least 24 h, preferably at least 30 h, and stable at 2-8 °C for at least 7 days, preferably at least 10 days.

The lyophilized powder is reconstituted into a suspension with a reconstitution medium. The reconstitution medium is selected from water for injection, a sodium chloride solution, and a glucose solution, preferably water for injection. The reconstituted suspension has a pH value of 3.4-5.8, preferably 3.6-5.6, or 3.8-5.0, and more preferably 3.9-4.8. The docetaxel albumin nanoparticle in the reconstituted suspension has a particle size of about 60-200 nm, preferably 90-150 nm, and more preferably 90-135 nm. The reconstituted suspension comprises 0-1.8% (w/v), preferably 0.45%-1.8% (w/v), and more preferably 0.9%-1.8% (w/v), of sodium chloride. The reconstituted isoosmotic suspension is stable at 25 °C for at least 24 h, preferably at least 30 h, and stable at 2-8 °C for at least 7 days, preferably at least 10 days.

In some embodiments, the composition comprising the docetaxel albumin nanoparticle described herein is stable at 25 °C for at least 36 months after lyophilization. The "stable" as used herein includes, but is not limited to, no significant degradation of docetaxel, no significant aggregation of protein nanoparticles, no significant increase in particle size, and no significant change in the docetaxel content, moisture, acidity, osmotic pressure or molar concentration, etc. The "stable" described herein can be one or more of the above-listed situations. In some embodiments, the "stable" means that there is no significant change in the content of 7-epidocetaxel and/or albumin aggregates.

In another aspect, the present disclosure further provides a drug prepared from the composition comprising the docetaxel albumin nanoparticle described above. The drug is in a clinically acceptable dosage form, preferably an injection, and further preferably a liquid injection or a lyophilized powder injection.

When the injection is a liquid injection, the liquid injection has a pH value of 3.4-5.8, preferably 3.6-5.6, or 3.8-5.0, and more preferably 3.9-4.8. The docetaxel albumin nanoparticle in the liquid injection has a particle size of about 60-200 nm, preferably 90-150 nm, and more preferably 90-135 nm; the liquid injection comprises 0-1.8% (w/v), preferably 0.45%-1.8% (w/v), more preferably 0.9%-1.8% (w/v), and more preferably 0.9% (w/v), of sodium chloride. In some embodiments, the liquid injection comprises 2-10 mg/mL, preferably 2-8 mg/mL of docetaxel. The liquid injection is stable at 25 °C for at least 24 h, preferably at least 30 h, and stable at 2-8 °C for at least 7 days, preferably at least 10 days. The "stable" as used herein means that there is no sedimentation of the nanoparticles or turbidity of the liquid injection.

When the injection is a lyophilized powder injection, the lyophilized powder injection comprises sodium chloride, and the sodium chloride and the docetaxel are in a weight ratio of (0.75-9):1, preferably (1-7):1, preferably (1.5-4.5):1, and most preferably 2.25:1. The docetaxel albumin nanoparticle has a particle size of about 60-200 nm, preferably 90-150 nm, and more preferably 90-135 nm. A suspension for preparing the lyophilized powder injection has a pH of 3.4-5.8, preferably 3.6-5.6, or 3.8-5.0, and more preferably 3.9-4.8 before lyophilization. The suspension is stable at 25 °C for at least 24 h, and stable at 2-8 °C for at least 10 days.

The lyophilized powder injection is reconstituted into a suspension with a reconstitution medium. The reconstitution medium is selected from water for injection, a sodium chloride solution, and a glucose solution, preferably water for injection. The resulting reconstituted suspension has a pH value of 3.4-5.8, preferably 3.6-5.6, or 3.8-5.0, and more preferably 3.9-4.8. The docetaxel albumin nanoparticle in the reconstituted suspension has a particle size of about 60-200 nm, preferably 90-150 nm, and more preferably 90-135 nm. The reconstituted suspension comprises 0-1.8% (w/v), preferably 0.45%-1.8% (w/v), more preferably 0.9%-1.8% (w/v), and much more preferably 0.9% of sodium chloride. In some embodiments, the reconstituted suspension comprises 2-10 mg/mL, preferably 2-8 mg/mL of docetaxel. The reconstituted suspension is stable at 25 °C for at least 24 h, preferably at least 30 h, and stable at 2-8 °C for at least 7 days, preferably at least 10 days.

The present disclosure further provides a method for preparing the composition, which comprises the following steps:
(1) dissolving docetaxel in an organic solvent to obtain an organic phase solution;
(2) taking a human serum albumin solution, and adding an acid to adjust the pH value to obtain an aqueous phase solution of an acid-denatured albumin; taking another salt solution;
(3) mixing the organic phase solution, the aqueous phase solution, and the salt solution for loading the drug to obtain a drug-loading solution; and
(4) dialyzing to obtain a dialyzed suspension;
wherein step (2) optionally comprises a step of diluting the human serum albumin solution with water for injection to obtain an albumin dilution before adding the acid to adjust the pH value. wherein, step (2) optionally comprises a step of incubating after adding the acid to adjust the pH value.

Wherein, step (3) optionally comprises a step of cooling after mixing for loading the drug. Wherein, step (4) optionally comprises concentrating the drug-loading solution obtained in step (3) to obtain a concentrated solution before dialyzing.

Wherein, step (4) optionally comprises a step of concentrating or diluting after dialyzing, so as to adjust the concentration of docetaxel in the dialyzed suspension.

Wherein, after step (4), the method optionally comprises step (5) of sterilizing and filtering. Wherein, after step (5), the method optionally comprises step (6) of lyophilizing.

Wherein, the docetaxel described in step (1) is in any form, preferably anhydrous docetaxel, docetaxel hemihydrate, or docetaxel trihydrate. The docetaxel (on an anhydrous basis) and the human serum albumin are in a mass ratio of 1:(2.0-10.0), preferably 1:(3.0-7.0), and more preferably 1:(4.0-6.0).

Wherein, the organic solvent described in step (1) is selected from a water-miscible solvent, such as ethanol, methanol, acetone, and dimethyl sulfoxide, etc., preferably ethanol. The organic phase solution comprises 45-90 mg/mL(on an anhydrous basis), preferably 45-70 mg/mL of docetaxel (on an anhydrous basis).

Wherein, the content of sodium caprylate in the human serum albumin solution described in step (2) is no more than 0.12 mmol/g protein, preferably no more than 0.08 mmol/g protein, and preferably 0.045-0.08 mmol/g protein.

Wherein, in step (2), the albumin dilution comprises 6-25 mg/mL, preferably 10-20 mg/mL, more preferably 12-18 mg/mL, and more preferably 15 mg/mL of albumin.

Wherein, in step (2), the acid is selected from an acidic amino acid or acidic polypeptide, an organic acid, and an inorganic acid. The acidic amino acid or acidic polypeptide includes, but is not limited to cysteine hydrochloride and glutathione; the organic acid includes, but is not limited to citric acid, tartaric acid, etc.; the inorganic acid includes, but is not limited to hydrochloric acid, sulfuric acid, etc. The acid is preferably cysteine hydrochloride, glutathione, or hydrochloric acid, and more preferably cysteine hydrochloride. The pH value is preferably 3.5-5.5, preferably 3.5-5.0, more preferably 3.8-4.7, and more preferably 4.0-4.5.

Wherein, in step (2), the incubating refers to heating to 35-42 °C, preferably 38-42 °C after adding the acid to adjust the pH value, and incubating for more than 30 min, preferably 30-60 min.

Wherein, the salt solution described in step (2) is selected from an aqueous solution of sodium chloride, potassium chloride, sodium sulfate, and magnesium sulfate, preferably an aqueous solution of sodium chloride; the salt solution is at a concentration of no less than 2%, preferably 2%-35%, and more preferably 10%-20%.

Wherein, in step (3), the drug loading is performed under a condition that the organic phase solution and the aqueous phase solution are heated to 35-42 °C, preferably 38-42 °C, and the three are mixed for loading the drug.

Wherein, the cooling described in step (3) refers to cooling to below room temperature, preferably 0-20 °C, and more preferably 7-15 °C.

Wherein, the concentrated solution described in step (4) comprises 4-10 mg/mL, preferably 6-10 mg/mL, more preferably 7-9 mg/mL, and more preferably 8 mg/mL, of docetaxel.

Wherein, the dialyzing in step (4) is to remove excessive small-molecule compounds. The type and amount of the dialysate and the molecular weight cutoff of the dialysis membrane are not particularly limited, and those skilled in the art can make a selection according to the general technical knowledge or experience. For the convenience of further formulation and clinical use, the dialysate is preferably an aqueous solution of a clinically acceptable tonicity adjusting agent, such as an aqueous solution of sodium chloride, glucose, phosphate, or citrate. In some embodiments, the dialyzing in step (4) is performed using a sodium chloride solution as a dialysate. The dialysis membrane has a molecular weight cutoff of 10-50 kDa, preferably 10-30 kDa, and more preferably 10 kDa or 30 kDa. The dialysate has a volume of no less than 3 times, preferably 3-10 times, and more preferably 3-6 times that of the drug-loading solution or the concentrated solution. The sodium chloride solution is at a concentration of no more than 1.8% (w/v), preferably 0.45%-1.8% (w/v), and more preferably 0.9%-1.8% (w/v).

In some embodiments, the content of sodium caprylate contained in the human serum albumin solution used in step (2) needs to be adjusted in advance. Those skilled in the art can select a suitable method to adjust the content of sodium caprylate in the human serum albumin solution according to the general technical knowledge or experience, including but not limited to dialysis. In some embodiments, the method for adjusting the content of sodium caprylate in a human serum albumin solution comprises:
diluting a commercially available human serum albumin solution with water for injection or normal saline to obtain a diluted albumin solution; dialyzing the diluted albumin solution with water for injection or normal saline as a dialysate, and partially removing sodium caprylate to obtain a human serum albumin solution with low sodium caprylate content.

In some embodiments, the human serum albumin solution is diluted with a dilution factor of no less than 4, preferably 4-7. The dialysis membrane in the dialysis has a molecular weight cutoff of 10-50 kDa, preferably 10-30 kDa, and more preferably 10 kDa or 30 kDa. The volume of the dialysate can be determined by those skilled in the art through routine tests according to the desired sodium caprylate content. Preferably, the dialysate has a volume of about more than 3 times, preferably 3-10 times, and more preferably 3-6 times that of the diluted albumin solution.

The human serum albumin solution with low sodium caprylate content comprises less than 0.16 mmol/g protein, preferably less than 0.12 mmol/g protein, preferably less than 0.10 mmol/g protein, and preferably less than 0.08 mmol/g protein, of sodium caprylate. The human serum albumin solution with low sodium caprylate content can be directly used for preparing the docetaxel albumin nanoparticle composition described herein, and can also mixed with a human serum albumin solution with an additional sodium caprylate content in proportion to obtain a desired content, and then used for preparing the docetaxel albumin nanoparticle composition.

In another aspect, the present disclosure further provides a composition comprising a docetaxel albumin nanoparticle, which is prepared by the above method.

In the preparation method, the dialysis step will remove excessive small-molecule compounds in the drug-loading solution or the concentrated solution. For example, in some embodiments, the acidic amino acid or acidic polypeptide is used in step (2) to adjust the pH for preparing the acid-denatured albumin; the dialysis step substantially removes excessive acidic amino acids or acidic polypeptides; the composition contains little free acidic amino acids or acidic polypeptides. The "containing little free acidic amino acids or acidic polypeptides" means that the content of free acidic amino acids or acidic polypeptides in the composition is less than 0.25% (w/w) of docetaxel. For example, in some embodiments, cysteine hydrochloride or glutathione is used to adjust the pH for preparing the acid-denatured albumin, and the content of free cysteine or glutathione in the composition is less than 0.25% (w/w) of docetaxel.

Furthermore, the present disclosure further provides a drug prepared from the composition comprising the docetaxel albumin nanoparticle prepared by the above method. The drug is in a clinically acceptable dosage form, preferably an injection, and further preferably a liquid injection or a lyophilized powder injection.

In another aspect, the present disclosure further provides a method for adjusting the content of sodium caprylate in a human serum albumin solution, comprising the following steps:
diluting a commercially available human serum albumin solution with water for injection or normal saline to obtain a diluted albumin solution; dialyzing the diluted albumin solution with water for injection or normal saline as a dialysate, and partially removing sodium caprylate to obtain a human serum albumin solution with low sodium caprylate content.

In some embodiments, the human serum albumin solution is diluted with a dilution factor of no less than 4, preferably 4-7. The dialysis membrane in the dialysis has a molecular weight cutoff of 10-50 kDa, preferably 10-30 kDa, and more preferably 10 kDa or 30 kDa. The volume of the dialysate can be determined by those skilled in the art through routine tests according to the desired sodium caprylate content. Preferably, the dialysate has a volume of about more than 3 times, preferably 3-10 times, and more preferably 3-6 times that of the diluted albumin solution.

The human serum albumin solution with low sodium caprylate content comprises less than 0.16 mmol/g protein, preferably less than 0.12 mmol/g protein, preferably less than 0.10 mmol/g protein, and preferably less than 0.08 mmol/g protein, of sodium caprylate.

The human serum albumin solution with low sodium caprylate content can be directly used for preparing the docetaxel albumin nanoparticle composition described herein, and can also mixed with a human serum albumin solution with an additional sodium caprylate content in proportion to obtain a desired content, and then used for preparing the docetaxel albumin nanoparticle composition.

The content of the docetaxel described herein is based on the anhydrous docetaxel.

The numerical values or numerical ranges described herein can fluctuate up and down within the range understood by those skilled in the art without affecting the implementation of the present disclosure, such as ±20%, or ±17%, or ±15%, or ±12%, or ±10%, or ±9%, or ±8%, or ±7%, or ±6%, or ±5%, or ±4%, or ±3%, or ±2%, or ±1%.

The expression "prepared from..." described herein is an open expression which does not exclude the presence of other optional components, for example, the expression "the composition is prepared from docetaxel and an acid-denatured albumin" should be understood as "the composition is prepared from a starting material composition comprising docetaxel and an acid-denatured albumin as starting materials" or "the composition is prepared from a starting material comprising docetaxel and an acid-denatured albumin as main components".

Surprisingly, in the present disclosure, the thermal stabilizer sodium caprylate in the albumin has a relatively great effect on the physical stability of the product. The reason is that sodium caprylate can compete with the drug for binding to the hydrophobic site of the albumin, which reduces the binding of the drug to the protein, resulting in instability of the nanosuspension. When a commercially available human serum albumin solution is directly used as an auxiliary material (containing 0.16 mmol/g protein of sodium caprylate), the sedimentation of nanoparticles occurs within 10 h. However, when the content of sodium caprylate in the albumin solution is reduced by means of dialysis to be lower than 0.08 mmol/g protein, the stability of the prepared nanoparticles is greatly improved, and the nanoparticles can maintain stable at 25 °C for at least more than 24 h, and can be stable at 2-8 °C for more than 10 days.

Secondly, the pH value is also an important factor affecting the physical stability of the docetaxel albumin nanoparticles. The pH values of the docetaxel albumin nanoparticle suspensions prepared according to the prior art are all above the isoelectric point of the albumin. In order to maintain the stability of the suspensions, a large amount of organic acids or salts thereof are required to be added as stabilizers, leading to relatively high osmotic pressure of the drug, causing obvious pain during clinical use, and causing osmotic damage to local cells and tissues after injection. In the present disclosure, the docetaxel albumin nanoparticle is prepared from an acid-denatured albumin, the content of sodium caprylate in the albumin solution is controlled, other salt stabilizers do not need to be added additionally, and a stable isoosmotic suspension is reconstituted, so that the stimulation to blood vessel is mild.

In addition, CN103054798A teaches that the stability of nanoparticles prepared from anhydrous docetaxel is significantly superior to that of nanoparticles prepared from docetaxel trihydrate and hemihydrate. However, in the technical schemes of the present disclosure, the water of crystallization of docetaxel, i.e., anhydrous, hemihydrate, or trihydrate, has no effect on the stability of the prepared docetaxel albumin nanoparticle composition. This greatly expands the options of the form of docetaxel and is valuable in industrial application.

The present disclosure provides a physically and chemically stable docetaxel albumin composition. Accelerated and long-term stability tests of the composition provided herein show that 7-epidocetaxel and protein polymers have minor change, and completed accelerated stability tests show that the lyophilized powder can be stably stored at 30 °C and 25 °C for 18 months, and the prediction of the existing data indicate that it can be stably stored at 30 °C for at least 20 months and at a temperature below 25 °C for at least 36 months . The completed static stability observation tests show that the composition provided herein can be stable for at least 24 h at room temperature and for at least 10 days under a refrigeration condition, and the turbidity of suspensions or the sedimentation of nanoparticles will not occur whether the composition is a suspension before lyophilization or a reconstituted suspension after lyophilization. The stability tests for longer periods of time are underway. Compared with the condition that the reconstituted suspension of the commercially available product is only stable for 8 h, the product of the present disclosure greatly decreases the clinical limitation. In addition, the docetaxel albumin composition provided herein has higher maximum tolerated dose (MTD) and lower toxicity than the commercially available docetaxel injection (TAXOTERE). Therefore, it is expected that the docetaxel albumin composition provided herein can improve the clinical dosage of docetaxel and improve the clinical safety.

### DETAILED DESCRIPTION

The following examples are specific illustrations of the present disclosure and should not limit the scope of the present disclosure.

Unless otherwise specified, the "stable" described in the examples means that no sedimentation of the nanoparticles or turbidity of the suspensions occurs.

Unless otherwise specified, the "reconstituted suspensions" described in the examples are isoosmotic suspensions.

### Example 1. Preparation of Composition of Docetaxel and Albumin

The formulations for docetaxel albumin nanoparticle compositions are as follows:

**Table 1. Formulations for docetaxel albumin nanoparticle compositions**

| Formulation No. | 1-1 | 1-2 | 1-3 | 1-4 |
|---|---|---|---|---|
| Anhydrous docetaxel | 8g | 8g | 8g | 8g |
| Human serum albumin | 36g | 36 g | 36 g | 80 g |
| Sodium chloride^{a} | 18g | 36 g | 9g | 18g |

| | | | | |
|---|---|---|---|---|
| Note: a refers to the content of sodium chloride in the pre-lyophilized suspension through calculation. The concentrations (w/v) of sodium chloride in the respective dialysates are: 0.9% in formulation 1-1, 1.8% in formulation 1-2, 0.45% in formulation 1-3, and 0.9% in formulation 1-4. | | | | |

(1) 8 g of anhydrous docetaxel was weighed and dissolved in 120 mL of ethanol to obtain an organic phase solution.
(2) A formulation amount of human serum albumin solution (with sodium caprylate content of 0.08 mmol/g protein) was diluted with water for injection to obtain a solution containing 15 mg/mL of albumin, then a proper amount of cysteine hydrochloride was added to adjust the pH to 4.1, and the mixed solution was incubated at 42 °C for 30 min to obtain an aqueous phase solution of acid-denatured albumin; sodium chloride solution was prepared at a concentration of 20% using water for injection.
(3) The organic phase solution and the aqueous phase solution were heated to 42 °C. The organic phase solution, the aqueous phase solution, and the salt solution were mixed for loading the drug, and the obtained material was cooled down to 15 °C in an ice-water bath to obtain a drug-loading solution.
(4) The drug-loading solution was concentrated until the concentration of docetaxel was about 8 mg/mL to obtain a concentrated solution; 5-fold dialysis was performed on the concentrated solution with a sodium chloride solution as a dialysate by using a dialysis membrane with a molecular weight cutoff of 30 kDa to obtain a dialyzed suspension; then a proper amount of the dialysate was added to adjust the concentration of the suspension to 4 mg/mL of docetaxel.
(5) The suspension was sterilized and filtered through a 0.45 µm + 0.2 µm membrane to obtain a pre-lyophilized suspension.
(6) The pre-lyophilized suspension obtained in step (5) was lyophilized to obtain a lyophilized powder.

The results show that the dialysis step can reduce the content of sodium caprylate. At the end of the dialysis step, the concentration of sodium chloride in the dialyzed suspension was substantially the same as the initial concentration of sodium chloride in the dialysate. The particle size of the nanoparticles in the suspension was detected by using a dynamic light scattering method, and the suspension was left to stand for observing the sedimentation. The results in Table 2 showed that the content of sodium chloride in the dialysate had no significant effect on the particle size of the docetaxel albumin nanoparticles in the pre-lyophilized suspension and in the reconstituted suspension. The pre-lyophilized suspension and the reconstituted suspension of each formulation were rested at 25 °C for 24 h, and no turbidity or precipitate appeared. The suspensions were also rested at 2-8 °C for 10 days, and no turbidity or precipitate appeared. It is suggested that the pre-lyophilized suspension and the reconstituted suspension of each formulation are stable at 25 °C for at least 24 h and stable at 2-8 °C for at least 10 days. The stability before and after lyophilization had no significant change.

As can be seen from the results comparison of formulations 1-1 and 1-4, the adjustment of the amount ratio of docetaxel to the albumin has no significant effect on the particle size of the nanoparticle and the stability of the suspension.

In addition, the inventors also detected the content of cysteine in the dialyzed suspension by high performance liquid chromatography with reference to General Rule 0512, Chinese Pharmacopoeia, Volume IV, 2020 Edition. The results show that the dialyzed suspension contains little free cysteine (the content is less than 0.25% (w/w) of docetaxel).

The inventors also detected the pH values of the suspension before and after dialysis and found that the pH values of the suspension before and after dialysis were substantially unchanged.

**Table 2. Particle size and stability of docetaxel albumin nanoparticles of different formulations**

| Formulation | | 1-1 | 1-2 | 1-3 | 1-4 |
|---|---|---|---|---|---|
| Pre-lyophilized suspension | Sodium caprylate (mmol/g protein) | 0.060 | 0.070 | 0.052 | 0.051 |
| | Particle size (nm) | 112 | 111 | 103 | 112 |
| | Stability (25 ± 2 °C) | Stable after 24 h | Stable after24 h | Stable after24 h | Stable after24 h |
| | Stability (2-8 °C) | Stable after 10 days | Stable after 10 days | Stable after 10 days | Stable after 1 0 days |
| Reconstituted suspension after lyophilization^{a} | Particle size (nm) | 112 | 115 | 104 | 113 |
| | Stability (25 ± 2 °C) | Stable after 24 h | Stable after24 h | Stable after24 h | Stable after24 h |
| | Stability (2-8 °C) | Stable after 10 days | Stable after 10 days | Stable after 10 days | Stable after 10 days |

| | | | | | |
|---|---|---|---|---|---|
| Note: a means that the lyophilized powder was reconstituted with water for injection into a suspension (isoosmotic suspension). Formulation 1-1 was reconstituted into an isoosmotic suspension comprising 4 mg/mL of docetaxel; formulation 1-2 was reconstituted into an isoosmotic suspension comprising 2 mg/mL of docetaxel; formulation 1-3 was reconstituted into an isoosmotic suspension comprising 8 mg/mL of docetaxel; formulation 1-4 was reconstituted into an isoosmotic suspension comprising 4 mg/mL of docetaxel. | | | | | |

### Example 2

Docetaxel trihydrate (8 g based on anhydrous docetaxel) was weighed and dissolved in 160 mL of absolute ethanol to obtain an organic phase solution. A human serum albumin solution containing 16 g of albumin (with sodium caprylate content of 0.08 mmol/g protein) was diluted with water for injection to obtain a solution containing 10 mg/mL of albumin, then a proper amount of cysteine hydrochloride was added to adjust the pH to 4.5, and the mixed solution was incubated at 40 °C for 1 h to obtain an aqueous phase solution of the acid-denatured albumin. Sodium chloride was prepared into a 10% salt solution with water for injection. The organic phase solution and the aqueous phase solution were heated to 40 °C, and then together mixed with the salt solution for loading the drug, and the obtained material was cooled down to 18 °C in an ice-water bath to obtain a drug-loading solution. The drug-loading solution was concentrated until the concentration of docetaxel was about 10 mg/mL to obtain a concentrated solution. 6-fold dialysis was performed on the concentrated solution with an isoosmotic sodium chloride solution (0.9%, w/v) as a dialysate by using a dialysis membrane with a molecular weight cutoff of 30 kDa. The dialyzed suspension was sterilized and filtered through a 0.45 µm + 0.2 µm membrane to obtain a pre-lyophilized suspension. The suspension was lyophilized to obtain a lyophilized powder.

In the pre-lyophilized suspension, the size of docetaxel albumin nanoparticle was 101.3 nm. After the suspension was rested at 25 °C for 24 h and at 2-8 °C for 10 days, no turbidity or precipitate appeared. The lyophilized powder was reconstituted with water for injection to obtain a reconstituted suspension (isoosmotic suspension), and the particle size of the docetaxel albumin nanoparticle had no significant change, which was 103.5 nm. The reconstituted suspension was rested 25 °C for 24 h and at 2-8 °C for 10 days, and no turbidity or precipitate appeared. It is suggested that the pre-lyophilized suspension and the reconstituted suspension are stable at 25 °C for at least 24 h and stable at 2-8 °C for at least 10 days.

The results as detected by high performance liquid chromatography showed that the dialyzed suspension contained little free cysteine (the content is about 0.13% (w/w) of docetaxel). The pH values of the suspension before and after dialysis were substantially unchanged.

### Example 3

Docetaxel hemihydrate (8 g based on anhydrous docetaxel) was weighed and dissolved in 80 mL of 96% ethanol to obtain an organic phase solution. A human serum albumin solution containing 50 g of albumin (with sodium caprylate content of 0.08 mmol/g protein) was diluted with water for injection to obtain a solution containing 20 mg/mL of albumin, and then a proper amount of cysteine hydrochloride was added to adjust the pH to 3.9 to obtain an aqueous phase solution of theacid-denatured albumin. Sodium chloride was prepared into a 2% salt solution with water for injection. The organic phase solution and the aqueous phase solution were heated to 35 °C. The organic phase solution, the aqueous phase solution, and the salt solution were mixed for loading the drug, and the obtained material was cooled down to 12 °C in an ice-water bath to obtain a drug-loading solution. The drug-loading solution was concentrated until the concentration of docetaxel was about 7 mg/mL to obtain a concentrated solution. 5-fold dialysis was performed on the concentrated solution with an isoosmotic sodium chloride solution (0.9%, w/v) as a dialysate by using a dialysis membrane with a molecular weight cutoff of 10 kDa. The dialyzed suspension was sterilized and filtered through a 0.45 µm + 0.2 µm membrane to obtain a pre-lyophilized suspension. The suspension was lyophilized to obtain a lyophilized powder.

In the pre-lyophilized suspension and the reconstituted suspension (isoosmotic suspension) obtained by reconstituting the lyophilized powder with water for injection, the particle sizes of the docetaxel albumin nanoparticles had no significant difference, which were 119.7 nm before lyophilization and 121.3 nm after reconstitution. The pre-lyophilized suspension and the reconstituted suspension were rested at 25 °C for 24 h and at 2-8 °C for 10 days, and no turbidity or precipitate appeared. It is suggested that the pre-lyophilized suspension and the reconstituted suspension are stable at 25 °C for at least 24 h and stable at 2-8 °C for at least 10 days. The results as detected by high performance liquid chromatography showed that the dialyzed suspension contained little free cysteine (the content is about 0.21% (w/w) of docetaxel). The pH values of the suspension before and after dialysis were substantially unchanged.

### Example 4. Effect of Forms of Docetaxel on Stability of Composition

Anhydrous docetaxel, docetaxel hemihydrate, and docetaxel trihydrate (all are 8 g based on the anhydrous docetaxel) were weighed and dissolved in 120 mL of ethanol, respectively, to obtain organic phase solutions. A human serum albumin solution containing 36 g of albumin (with sodium caprylate content of 0.08 mmol/g protein) was diluted with water for injection to obtain a solution containing 20 mg/mL of albumin, then a proper amount of cysteine hydrochloride was added to adjust the pH to 4.3, and the mixed solution was incubated at 42 °C for 60 min to obtain an aqueous phase solution of acid-denatured albumin. Sodium chloride was prepared into a 10% salt solution with water for injection. The organic phase solution and the aqueous phase solution were heated to 42 °C and then together mixed with the salt solution for loading the drug, and the obtained material was cooled down to 12 °C in an ice-water bath to obtain a drug-loading solution. The drug-loading solution was concentrated until the concentration of docetaxel was about 7 mg/mL to obtain a concentrated solution. 5-fold dialysis was performed on the concentrated solution with an isoosmotic sodium chloride solution as a dialysate by using a dialysis membrane with a molecular weight cutoff of 30 kDa. The dialyzed suspension was sterilized and filtered through a 0.45 µm + 0.2 µm membrane to obtain a pre-lyophilized suspension. The suspension was lyophilized to obtain a lyophilized powder.

As shown in Table 3 below, the docetaxel albumin nanoparticles were prepared by the same method using anhydrous docetaxel, docetaxel hemihydrate, and docetaxel trihydrate as starting materials, and the particle sizes of the obtained nanoparticles had no significant difference, all of which were around 110 nm, and did not significantly change before and after lyophilization. In addition, the stability of the pre-lyophilized suspension and the reconstituted suspension (containing 4 mg/mL of docetaxel) obtained by reconstituting the lyophilized powder with water for injection also had no significant difference. The suspensions were rested at 25 °C for 24 h and at 2-8 °C for 10 days, and no turbidity or precipitate appeared. It is suggested that the pre-lyophilized suspension and the reconstituted suspension described above can be stable at 25 °C for at least 24 h and stable at 2-8 °C for at least 10 days. It can be seen that whether docetaxel is anhydrous or hydrated does not affect the implementation of the present disclosure.

The results as detected by high performance liquid chromatography showed that the dialyzed suspension contained little free cysteine (the content is less than 0.25% (w/w) of docetaxel).

**Table 3. Effect of use form of docetaxel on stability of composition**

| | Pre-lyophilized suspension | | | Reconstituted suspension after lyophilization | | |
|---|---|---|---|---|---|---|
| | Particle size (nm) | Stability (25 ± 2 °C) | Stability (2-8 °C) | Particle size (nm) | Stability (25 ± 2 °C) | Stability (2-8 °C) |
| Anhydrous docetaxel | 112.3 | Stable after 24 h | Stable afterl0 days | 110.9 | Stable after24 h | Stable after 10 days |
| Docetaxel hemihydrate | 108.2 | Stable after24 h | Stable after 10 days | 107.9 | Stable after24 h | Stable after10 days |
| Docetaxel trihydrate | 109.1 | Stable after24 h | Stable after 10 days | 110.5 | Stable after24 h | Stable after 10 days |

### Example 5. Chemical Stability Investigation

The lyophilized powder comprising the docetaxel albumin nanoparticles obtained from formulation 1-1 in Example 1 was placed in a vial, and a rubber stopper and an aluminum cap were pressed. The vials were stored for 18 months under different storage conditions. The percentage content of 7-epidocetaxel and the relationship between the protein polymer content and time in the product were investigated. The results are shown in Table 4.

**Table 4. Product stability of formula 1-1 in Example 1**

| Storage conditions | 7-Epidocetaxel content (%) | | | Protein polymer content (%) | | |
|---|---|---|---|---|---|---|
| | 0 days | 12 months | 18 months | 0 days | 12 months | 18 months |
| 30 ± 2°C, 65% ± 5% RH | 0.07 | 0.56 | 0.79 | 3.8 | 3.4 | 3.6 |
| 25 ± 2°C, 60% ± 10% RH | 0.07 | 0.41 | 0.42 | 3.8 | 3.3 | 3.6 |
| 2-8°C | 0.07 | 0.14 | N/A* | 3.8 | 3.5 | N/A* |

| | | | | | | |
|---|---|---|---|---|---|---|
| Note: the acceptable limit for defining the stability of the pharmaceutical composition is "the percentage content of 7-epidocetaxel ≤ 1.0%". N/A* indicates that the experiment has not reached this time point and thus no data are available. | | | | | | |

The results in Table 4 showed that the production of 7-epidocetaxel was associated with the storage temperature. The higher the storage temperature was, the faster the content of 7-epidocetaxel increased. The product of the present disclosure can effectively control the production of 7-epidocetaxel. The results in Table 4 showed that under the three test conditions described above, the content of 7-epidocetaxel in the product of the present disclosure was in a controllable range (content ≤ 1.0%), and the protein polymer content had no significant change. It is predicted from the existing data that the product of the present disclosure can be stably stored at 30 °C for at least 20 months and at a temperature below 25 °C for at least 36 months.

When the API starting material was docetaxel in other forms, such as hemihydrate and trihydrate, the change in chemical stability (the percentage content of 7-epidocetaxel and the protein polymer content) was similar to that of anhydrous docetaxel as the starting material under the test conditions described above.

In the Chinese Patent CN106137969A, arginine, proline, and the like were added as inhibitors in the formulations, and the effect of arginine as an inhibitor was considered to be the best. The product could be stored at 2-8 °C for at least 24 months, even up to 30 months. The data provided by this patent showed that the content of 7-epidocetaxel was between 0.5% and 0.61% when the product was stored at 2-8 °C for 12 months. However, in the present disclosure, the content of 7-epidocetaxel was only about 0.41% when the product was stored at 25 °C for 12 months, and the content of 7-epidocetaxel was only 0.14% when the product was stored at 2-8 °C for 12 months, which is enough to show that the chemical stability of docetaxel can be more effectively ensured in the present disclosure compared with CN106137969A.

### Example 6. Effect of Sodium Caprylate Content on Stability of The Composition

8 g of anhydrous docetaxel was weighed and dissolved in 120 mL of ethanol to obtain an organic phase solution.

Human serum albumin solutions (containing 36 g of human serum albumin) containing sodium caprylate at different concentrations were diluted with water for injection, respectively, to obtain solutions containing 15 mg/mL of albumin, then a proper amount of cysteine hydrochloride was added to adjust the pH to 4.2, and the mixed solution was incubated at 42 °C for 30 min to obtain an aqueous phase solution of acid-denatured albumin. Sodium chloride was prepared into a 15% salt solution with water for injection.

The organic phase solution and the aqueous phase solution were heated to 42 °C. The organic phase solution, the aqueous phase solution, and the salt solution were mixed for loading the drug, and the obtained material was cooled down to 15 °C in an ice-water bath to obtain a drug-loading solution. The drug-loading solution was concentrated until the concentration of docetaxel was about 8 mg/mL to obtain a concentrated solution. 5-fold dialysis was performed on the concentrated solution with an isoosmotic sodium chloride solution as a dialysate (using a dialysis membrane with a molecular weight cutoff of 30 kDa). The dialyzed suspension was sterilized and filtered through a 0.45 µm + 0.2 µm membrane to obtain a pre-lyophilized suspension. The suspension was lyophilized to obtain a lyophilized powder.

The results in Table 5 showed that the content of sodium caprylate in the starting materials had no significant effect on the particle size of the docetaxel albumin nanoparticle. However, for the docetaxel albumin nanoparticle prepared directly from a commercially available human serum albumin solution (containing 0.16 mmol/g protein of sodium caprylate) as a starting material, the turbidity appeared after 10 h at 25 °. The physical stability of the pre-lyophilized suspension and the reconstituted isoosmotic suspension was poor. Reducing the content of sodium caprylate in the human serum albumin solution is helpful to improve the physical stability of the nanoparticle suspension. When the content of sodium caprylate in the human serum albumin solution was less than 0.08 mmol/g protein, the solution could maintain a stable suspending state at 25 °C for more than 24 h, and the stability time was more than 2 times that of a solution containing 0.16 mmol/g protein of sodium caprylate. When the content of sodium caprylate in the human serum albumin solution was less than 0.08 mmol/g protein, the pre-lyophilized suspension and the reconstituted isoosmotic suspension could be stable at 2-8 °C for at least more than 10 days.

**Table 5. Effect of sodium caprylate content on stability of composition**

| Sodium caprylate content in the starting material (mmol/g protein) | Pre-lyophilized suspension | | | | Reconstituted suspension with the lyophilized powder^{a} | | |
|---|---|---|---|---|---|---|---|
| | Sodium caprylate content (mmol/g protein) | Particle size (nm) | Stability (25 ± 2 °C) | Stability (2-8 °C) | Particle size (nm) | Stability (25 ± 2 °C) | Stability (2-8 °C) |
| 0.045 | 0.042 | 100.2 | Still no sedimentation or turbidity after24 h | Stable after standing for 10 days | 101.3 | Still no sedimentation or turbidity after standing for 24 h | Stable after standing for 10 days |
| 0.06 | 0.056 | 97.23 | Still no sedimentation or turbidity afte 24 h | Stable after 10 days | 98.12 | Still no sedimentation or turbidity afte 24 h | Stable afte 10 days |
| 0.08 | 0.070 | 96.94 | Still no sedimentation or turbidity afte 24 h | Stable after 10 days | 98.02 | Still no sedimentation or turbidity afte 24 h | Stable after 10 days |
| 0.12 | 0.072 | 101.5 | Turbidity appeared after 18 h | Not investigated | 100.2 | Turbidity appeared after 18 h | Not investigated |
| 0.16 | 0.075 | 102.8 | Turbidity appeared afterlO h | Not investigated | 103.5 | Turbidity appeared after 10 h | Not investigated |

| | | | | | | | |
|---|---|---|---|---|---|---|---|
| Note: a means that the lyophilized powder was reconstituted with water for injection to obtain a reconstituted suspension (isoosmotic suspension). | | | | | | | |

### Example 7. Effect of pH Value of Aqueous Phase Solution on Stability

The effect of the pH value of the aqueous phase solution on the stability of the product was compared. The process formulation and preparation method were referred to formulation 1-1 in Example 1. Only in step (2), pH of the aqueous phase solution was adjusted to 7.0, 6.5, 6.0, 5.5, 5.0, 4.7, 4.1, 3.8, and 3.5, respectively, using cysteine hydrochloride. The particle size of the nanoparticles in the pre-lyophilized suspension and in the reconstituted suspension and the change in the stability of the suspensions were observed.

It was found that when the pH of the aqueous phase solution was in the range of 3.5-5.5, the particle size of the obtained nanoparticles was slightly increased along with the increase of the pH, but the lyophilization and reconstitution had no significant effect on the particle size. When the pH of the aqueous phase solution was in the range of 3.5-5.5, the obtained pre-lyophilized suspension and the reconstituted suspension could both maintain a stable suspending state at 25 °C for more than 20 h and at 2-8 °C for at least 7 days. Further, when the pH of the aqueous phase solution was in the range of 3.8-4.7, the stable suspending state could be still maintained after the pre-lyophilized suspension and the reconstituted suspension were rested at 25 °C for 30 h and at 2-8 °C for 10 days. The lyophilization and reconstitution had no significant effect on the stability.

However, when the pH of the aqueous phase solution was more than 6.0, the particle size of the obtained nanoparticles was significantly increased. When the pH was 6.0, the particle size of the nanoparticles was increased to 180 nm. In addition, when the pH of the aqueous phase solution was above 6.0, the stability of the suspension was significantly reduced, the turbidity appeared within 1 h, and the stable suspending state of the nanoparticles could not be maintained.

**Table 6. Effect of pH of aqueous phase protein on docetaxel albumin nanoparticles**

| pH of aqueous phase solution | Pre-lyophilized suspension | | | | Reconstituted suspension after lyophilization^{a} | | |
|---|---|---|---|---|---|---|---|
| | pH | Particle size (nm) | Stability (25 ± 2 °C) | Stability (2-8 °C) | Particle size (nm) | Stability (25 ± 2 °C) | Stability (2-8 °C) |
| 3.5 | 3.6 | 92 | Stable after20 h | Stable after? days | 93 | Stable after20 h | Stable after? days |
| 3.8 | 3.9 | 95 | Stable after30 h | Stable after 10 days | 94 | Stable after30 h | Stable after 10 days |
| 4.1 | 4.3 | 98 | Stable after 30 h | Stable after 10 days | 100 | Stable after 30 h | Stable after 10 days |
| 4.7 | 4.8 | 97 | Stable after30 h | Stable after 10 days | 96 | Stable after 30 h | Stable after 10 days |
| 5.0 | 5.1 | 114 | Stable after 20 h | Stable after? days | 117 | Stable after20 h | Stable after? days |
| 5.5 | 5.6 | 112 | Stable after20 h | Stable after? days | 114 | Stable after20 h | Stable after? days |
| 6.0 | Not investigated | 180 | Turbidity appeared after1 h | Not investigated | Not investigated | Not investigated | Not investigated |
| 6.5 | Not investigated | Not investigated | Turbidity appeared after30 min | Not investigated | Not investigated | Not investigated | Not investigated |
| 7.0 | Not investigated | Not investigated | Turbidity appeared immediately | Not investigated | Not investigated | Not investigated | Not investigated |

| | | | | | | | |
|---|---|---|---|---|---|---|---|
| Note: a means that the lyophilized powder was reconstituted with water for injection to obtain a reconstituted suspension (isoosmotic suspension). | | | | | | | |

### Example 8. Preparation of Human Serum Albumin Solution with Low Sodium Caprylate Content

### Example 8-1

A commercially available human serum albumin solution (with sodium caprylate content of 0.16 mmol/g protein) was diluted with water for injection to obtain an albumin dilution containing 15 mg/mL of albumin. 6-fold dialysis was performed on the albumin dilution with water for injection as a dialysate (using a membrane with a molecular weight cutoff of 30 kDa), and sodium caprylate in the protein was partially removed to obtain a human serum albumin solution with low sodium caprylate content.

The content of sodium caprylate in the obtained protein solution was detected with reference to the method for determining sodium caprylate in the human serum albumin solution recorded in Chinese Pharmacopoeia (3111, Volume IV, 2020 Edition), and the results showed that the treated protein solution contained about 0.045 mmol/g protein of sodium caprylate.

### Example 8-2

A commercially available human serum albumin solution (with sodium caprylate content of 0.16 mmol/g protein) was diluted with normal saline to obtain an albumin dilution containing 12 mg/mL of albumin. 5-fold dialysis was performed on the albumin dilution with normal saline as a dialysate (using a membrane with a molecular weight cutoff of 10 kDa), and sodium caprylate in the protein was partially removed to obtain a human serum albumin solution with low sodium caprylate content. The detection showed that the obtained human serum albumin solution with low sodium caprylate content contained about 0.060 mmol/g protein of sodium caprylate.

### Example 8-3

A commercially available human serum albumin solution (with sodium caprylate content of 0.16 mmol/g protein) was diluted with water for injection to obtain an albumin dilution containing 20 mg/mL of albumin. 3-fold dialysis was performed on the albumin dilution with water for injection as a dialysate (using a membrane with a molecular weight cutoff of 30 kDa), and sodium caprylate in the protein was partially removed to obtain a human serum albumin solution with low sodium caprylate content. The detection showed that the obtained human serum albumin solution with low sodium caprylate content contained about 0.080 mmol/g protein of sodium caprylate.

The human serum albumin solutions with low sodium caprylate content in Examples 8-1 to 8-3 above could be further concentrated for the convenience of storage or application.

### Example 9. Effect of Process on Secondary Structure of Protein

A drug-loading nanosuspension and a blank suspension (in step (1), the drug docetaxel was not added) were prepared according to the formulations and preparation method in formulation 1-1 in Example 1, and the ratios of each secondary structure of the protein in the human serum albumin solution, the blank suspension, and the drug-loading nanosuspension were investigated by circular dichroism approach. The results are shown in Table 7.

The results show that the blank suspension and the drug-loading nanosuspension prepared through the whole process flow have differences compared with the human serum albumin solution in terms of the secondary structures of protein, α-helix, β-sheet, turn, and random coil, indicating that the process changes the secondary structures of albumin.

The research results indicate that the secondary structures of the acid-denatured albumin cannot be restored to the pre-denatured state even if the acid is removed in the dialysis step in the present disclosure. In addition, after docetaxel is bound, the secondary structures of the drug-loading albumin are further changed relative to the acid-denatured albumin without loading a drug.

**Table 7. Ratios of secondary structures of protein**

| | Human serum albumin solution | Blank suspension^{a} | Drug-loading nanosuspension^{a} |
|---|---|---|---|
| α-helix (%) | 39.5 | 23.9 | 27.9 |
| β-sheet (%) | 15.1 | 11.7 | 4.4 |
| Turn (%) | 16.8 | 26.2 | 30.1 |
| Random coil (%) | 28.6 | 38.2 | 37.6 |

| | | | |
|---|---|---|---|
| Note: a: both the blank suspension and the drug-loading nanosuspension described in Example 9 were isoosmotic suspensions that were reconstituted after lyophilization. | | | |

### Example 10. Preparation with Reference to Example 1 in Patent CN 106137969 B (201510157393.1)

1.5 g of anhydrous docetaxel was weighed and ultrasonically dissolved in 100 mL of absolute ethanol to obtain an oil phase solution. A human serum albumin solution containing 7.5 g of albumin (at a concentration of 200 mg/mL) was diluted with water for injection to form a solution containing 6 mg/mL of albumin, and the volume of the aqueous phase was 1250 mL in total. 500 mgL-glutathione was added to the aqueous phase, and the mixed solution was incubated at 70 °C for 6 min. The oil phase was dispersed homogeneously into the aqueous phase under a high shear rate of 1000 rpm to obtain a suspension.

The suspension was white without opalescence, visual turbid, and the precipitates appeared after 10 min, then the subsequent operations in the patent could not perform as a result that the technicians considered a poor stability of the obtained suspension. Therefore, the inventors believed that the stability of the product of the present disclosure is sufficiently proved to be significantly superior to that of the product in the patent CN 106137969 B by comparing the storage stability data of the product prepared by the method in the present disclosure with the storage stability data provided in the patent.

| Sample/storage conditions | | Related substance% (7-epidocetaxel) | | | | | |
|---|---|---|---|---|---|---|---|
| | | 0 day | 3 months | 6 months | 12 months | 24 months | 30 months |
| Example 1 of the present disclosure | 25±2 °C, 60%±5%RH | 0.07 | 0.22 | 0.22 | 0.41 | 0.60 | N/A |
| Example 1 of the present disclosure | 2-8 °C | 0.07 | N/A | N/A | 0.14 | 0.24 | N/A |
| Example 1-d of patent CN 106137969 B | 2-8 °C | 0.21 | 0.25 | 0.28 | 0.51 | 0.62 | 0.75 |

| | | | | | | | |
|---|---|---|---|---|---|---|---|
| **N/A: this time point was not available in the stability study** | | | | | | | |

### Example 11. Investigation of Effect of Protein-Drug Ratio of 1.5 (While Reducing Protein Concentration to 5 mg/mL)

1.6 g of anhydrous docetaxel was weighed and dissolved in 23.7 mL of absolute ethanol to obtain an organic phase solution at a concentration of 67.5 mg/mL. A human serum albumin solution containing 2.4 g of albumin (with sodium caprylate content of 0.08 mmol/g protein) was diluted with water for injection to obtain a solution containing 5 mg/mL of albumin, then a proper amount of cysteine hydrochloride was added to adjust the pH to 4.0, and the mixed solution was incubated at 40 °C for 0.5 h to obtain an aqueous phase solution of acid-denatured albumin. Sodium chloride was prepared into a 14.4% salt solution with water for injection. The organic phase solution and the aqueous phase solution were heated to 40 °C, and then together mixed with the salt solution for loading the drug, and the obtained material was cooled down to 18 °C in an ice-water bath to obtain a drug-loading solution. The drug-loading solution was concentrated until the concentration of docetaxel was about 6 mg/mL to obtain a concentrated solution. 5-fold dialysis was performed on the concentrated solution with an isoosmotic sodium chloride solution (0.9%, w/v) as a dialysate by using a dialysis membrane with a molecular weight cutoff of 30 kDa. The dialyzed suspension was sterilized and filtered through a 0.45 µm + 0.2 µm membrane to obtain a pre-lyophilized suspension. The suspension was lyophilized to obtain a lyophilized powder.

In the pre-lyophilized suspension, the docetaxel albumin nanoparticle had a particle size of 91.38 nm. After 20 h at 25 °, the suspension was completely turbid. The lyophilized powder was reconstituted with water for injection to obtain a reconstituted suspension (isoosmotic suspension) in which the turbidity was observed visually.

### Example 12. Investigation of Effects of Different Dialysates

1.8 g of anhydrous docetaxel was weighed and dissolved in 26.7 mL of absolute ethanol to obtain an organic phase solution at a concentration of 67.5 mg/mL. A human serum albumin solution containing 8.1 g of albumin (with sodium caprylate content of 0.08 mmol/g protein) was diluted with water for injection to obtain a solution containing 15 mg/mL of albumin, then a proper amount of cysteine hydrochloride was added to adjust the pH to 4.0, and the mixed solution was incubated at 40 °C for 0.5 h to obtain an aqueous phase solution of acid-denatured albumin. Sodium chloride was prepared into a 14.4% salt solution with water for injection. The organic phase solution and the aqueous phase solution were heated to 40 °C, and then together mixed with the salt solution for loading the drug, and the obtained material was cooled down to 18 °C in an ice-water bath to obtain a drug-loading solution. The drug-loading solution was concentrated until the concentration of docetaxel was about 6 mg/mL to obtain a concentrated solution.
(1) 5-fold dialysis was performed on the concentrated solution with an isoosmotic glucose solution (5%, w/v) as a dialysate by using a dialysis membrane with a molecular weight cutoff of 30 kDa. The dialyzed suspension was sterilized and filtered through a 0.45 µm + 0.2 µm membrane to obtain a pre-lyophilized suspension. The suspension was lyophilized to obtain a lyophilized powder.
   In the pre-lyophilized suspension, the docetaxel albumin nanoparticle had a particle size of 102.2 nm. After 16 h at 25 °C, relatively more precipitates were left at the bottom in the suspension. After 16 h at 2-8 °C, the light transmittance was reduced. The lyophilized powder was reconstituted with water for injection to obtain a reconstituted suspension (isoosmotic suspension), and the particle size of the docetaxel albumin nanoparticle had no significant change, which was 103.3 nm. The reconstituted suspension was turbid after resting at 25 °C for 2.5 h.
(2) 5-fold dialysis was performed on the concentrated solution with an isoosmotic PBS buffer (with a pH of 7.31 and an osmotic pressure of 320 mOsmol/Kg) as a dialysate by using a dialysis membrane with a molecular weight cutoff of 30 kDa. The dialyzed suspension was sterilized and filtered through a 0.45 µm + 0.2 µm membrane to obtain a pre-lyophilized suspension. The suspension was lyophilized to obtain a lyophilized powder.

In the pre-lyophilized suspension, the docetaxel albumin nanoparticle had a particle size of 92.43 nm. After the suspension was rested at 25 °C for 16 h, a few precipitates appeared at the bottom. After the suspension was left to stand at 2-8 °C for 24 h, the light transmittance was reduced. The lyophilized powder was reconstituted with water for injection to obtain a reconstituted suspension (isoosmotic suspension), and the particle size of the docetaxel albumin nanoparticle had no significant change, which was 90.91 nm. The precipitates could be found in the reconstituted suspension after 2.5 h at 25 °C phase.

### Example 13. Maximum Tolerated Dose (MTD) and Toxicity Comparison of Docetaxel for Injection (Albumin-Bound, DTX-HSA) and Docetaxel Injection (TAXOTERE) in Nude Mice

### 1. Drugs and test materials

### 1.1 Test sample

Docetaxel for injection (albumin-bound, DTX-HSA) was prepared by the preparation method in Example 1 using formulation 1-1 in Example 1 of the present application.

### 1.2 Control drug

Docetaxel injection (trade name: TAXOTERE)

| | | | |
|---|---|---|---|
| Abbreviation | TAXOTERE | Specification | 0.5 mL:20 mg |
| Drug characteristics | Yellow to brown viscous liquid with a solvent | Content | 10 mg/mL after dilution of a special solvent |
| Batch No. | 7F163B | Storage condition | Stored at 2-25 °C in the dark |
| Supplier | Sanofi (Hangzhou) Pharmaceutical Co., Ltd. | | |

### 2. Experimental animals

| | | | |
|---|---|---|---|
| Animal species | NU/NU | Grade | SPF grade |
| Gender/number | Female/50 mice, 40 mice actually used. | Mouse age/body weight | 4-6 weeks/19-23 g |
| Purchase time | 2019.04.10 | Animal certification No. | 1100111911002464 |
| Animal production license No. | SCXK (Beijing) 2016-0006 | Supplier | Beijing Vital River Laboratory Animal Technology Co., Ltd. |

### 3. Experimental design

### 3.1 Experimental principle

Definition of the maximum tolerated dose (MTD) of the drug for intravenous administration to the mice in the present test: If no death and no irreversible toxic reactions, or no weight loss of more than 15% for 3 consecutive days was found during the observation period, this dose was considered as the maximum tolerated dose for an acute single administration.

### 3.2 Administration dose setting

The isoosmotic concentration of DTX-HSA after reconstituting was 3.801 mg/mL, and the maximum administration volume of the slow intravenous injection to the mice was 25 mL/kg according to the guidelines on the allowable administration volume and blood collection volume for different routes of administration or blood collection in animals jointly issued by the European Federation of Pharmaceutical Industries Associations and the European Center for Validation of Alternative Methods in 2001. The drugs were administered 3 times within 24 h, and the maximum dose could be 285.1 mg/kg.

Pre-selected doses in this experiment were:
docetaxel for injection (albumin-bound): 285.1, 228.1, 182.5, and 146.0 mg/kg (gradient 1.25); and
docetaxel injection (TAXOTERE): 187.5, 150, 120, and 96 mg/kg (gradient 1.25).

### 4. Experimental method

### 4.1 Animal grouping and labeling

Healthy mice with relatively small differences in body weight were selected and divided into 8 groups with 5 mice in each group based on weight in a balanced manner, i.e., DTX-HSA 285.1, 228.1, 182.5, and 146.0 mg/kg groups and TAXOTERE 187.5, 150, 120, and 96 mg/kg groups.

### 4.2 Administration

Route of administration: intravenous injection
Frequency of administration: 3 times within 24 h, with an interval of 4 h.
Administration volume: 25 mL/kg.
Administration speed: slow.
Dose of administration: the dose of administration was calculated according to the latest weighing.

### 4.3 Index observations

General state observation: all animals were observed every day during the experiment period, and the observation indexes included, but were not limited to, skin, hair, eyes, ears, nose, oral cavity, chest, abdomen, urogenital area, limbs, and other sites, as well as changes in breath, movement, urination, defecation, and behaviors. Adverse effects of the animals are shown in the table below. Body weight: all animals were weighed once before the experiment and the animals having an appropriate body weight were selected for the experiment. The animals were weighed once every day at a fixed time.

Death and moribundity: the time of death was recorded for the dead animals. The frequency of observation was increased for the moribund animals and the time of death was determined during the experiment.

### 4.4 Evaluation indexes

The maximum dose, without death, irreversible toxic symptoms, or weight loss of more than 15% for 3 consecutive days founded in the mice during the observation period, was considered as the maximum tolerated dose (MTD) of the drug in the experiment.

### 5. Experimental results

### 5.1 Mortality

No animal death was observed in the DTX-HSA and TAXOTERE administration groups.

### 5.2 Clinical observations

Animals in DTX-HSA 285.1 and 228.1 mg/kg groups could be observed to have mild hind limb tremors starting from D4 and D7, respectively. The animals in 228.1 mg/kg group recovered from the hind limb tremor symptom on D20, and the animals in 285.1 mg/kg group recovered from the hind limb tremor symptom on D22. No significant abnormality was observed in animals in DTX-HSA 182.5 and 146.0 mg/kg groups.

Animals in TAXOTERE 187.5 and 150 mg/kg groups could be observed to have mild-to-moderate hind limb tremors starting from D4 and D6, respectively. The animals in 150 mg/kg group recovered from the hind limb tremor symptom on D19, and the animals in 187.5 mg/kg group recovered from the hind limb tremor symptom on D24. No significant abnormality was observed in animals in TAXOTERE 120 and 96 mg/kg groups.

### 5.3 Body weight

1/5 animals in DTX-HSA 285.1 mg/kg group had a weight loss of more than 15% during D5-D8, 1/5 animals had a weight loss of more than 15% on D10, D12, and D13, and the animals with a weight loss of more than 15% were not found in other DTX-HSA administration groups.

1/5 animals in TAXOTERE 187.5 mg/kg group had a weight loss of more than 15% during D4-D14 and the animals with a weight loss of more than 15% were not found in other TAXOTERE administration groups.

### Toxicity in mice after administration (n = 5)

| Drug | Dose (mg/kg) | Number of dead animals | Number of animals with > 15% weight loss for 3 consecutive days | Symptom | MTD value (mg/kg) | Weight gain % on D24 |
|---|---|---|---|---|---|---|
| DTX-HSA | 285.1 | 0 | 1(D5~D8) | Animals exhibited various degrees of hind limb tremors during D4-D22. | 228.1 (76.0×3) | 14.6 |
| | 228.1 | 0 | 0 | Animals exhibited various degrees of hind limb tremors during D7-D20. | | 15.1 |
| | 182.5 | 0 | 0 | No significant abnormality. | | 13.1 |
| | 146.0 | 0 | 0 | No significant abnormality. | | 16.2 |
| TAXOTERE | 187.5 | 0 | 1 (D4-D 14) | Animals exhibited various degrees of hind limb tremors during D4-D24. | 150.0 (50.0×3) | 6.2 |
| | 150.0 | 0 | 0 | Animals exhibited various degrees of hind limb tremors during D6-D19. | | 13.8 |
| | 120.0 | 0 | 0 | No significant abnormality. | | 14.1 |
| | 96.0 | 0 | 0 | No significant abnormality. | | 17.4 |

| | | | | | | |
|---|---|---|---|---|---|---|
| MTD: a maximum dose if no death, irreversible toxic symptoms, or weight loss of more than 15% for 3 consecutive days was found in the mice during the observation period. Observation time: 24 days. | | | | | | |

### 6. Conclusion

Under the experimental conditions, the MTD of the DTX-HSA in nude mice is 228.1 mg/kg; the MTD of the TAXOTERE in nude mice is 150.0 mg/kg.

## Claims

1. A composition, comprising a docetaxel albumin nanoparticle, wherein the composition comprises docetaxel and an acid-denatured albumin; the docetaxel is preferably anhydrous docetaxel, docetaxel hemihydrate, or docetaxel trihydrate.

2. A composition, comprising a docetaxel albumin nanoparticle, wherein the composition is prepared from docetaxel and an acid-denatured albumin; the docetaxel is preferably anhydrous docetaxel, docetaxel hemihydrate, or docetaxel trihydrate.

3. The composition according to claim 1 or 2, wherein the acid-denatured albumin is obtained by adding an acid to human serum albumin to adjust to an appropriate pH value and then denaturing; preferably:
(1) the acid is selected from an acidic amino acid or acidic polypeptide, an organic acid, and an inorganic acid; the acidic amino acid or acidic polypeptide comprises cysteine hydrochloride and glutathione; the organic acid comprises citric acid and tartaric acid; the inorganic acid comprises hydrochloric acid and sulfuric acid; the acid is preferably cysteine hydrochloride, glutathione, or hydrochloric acid, and more preferably cysteine hydrochloride; and/or
(2) the appropriate pH value is preferably 3.5-5.5, preferably 3.5-5.0, more preferably 3.8-4.7, and more preferably 4.0-4.5; and/or
(3) the content of sodium caprylate in the human serum albumin is no more than 0.08 mmol/g protein, preferably 0.03-0.08 mmol/g protein, further preferably 0.04-0.08 mmol/g protein, and more preferably 0.04-0.07 mmol/g protein; and/or
(4) the docetaxel (on an anhydrous basis) and the human serum albumin are in a mass ratio of 1:(2.0-10.0), preferably 1 :(3.0-7.0), and more preferably 1:(4.0-6.0).

4. The composition according to claim 1 or 2, wherein the docetaxel albumin nanoparticle has a particle size of 60-200 nm, preferably 90-150 nm, and more preferably 90-135 nm.

5. The composition according to claim 1 or 2, wherein the composition optionally comprises a tonicity adjusting agent; the tonicity adjusting agent is selected from sodium chloride, glucose, phosphate, and citrate; preferably, the tonicity adjusting agent is sodium chloride, and the sodium chloride and the docetaxel are in a weight ratio of (0.75-9):1, preferably (1-7):1, preferably (1.5-4.5):1, and most preferably 2.25:1.

6. The composition according to claim 1 or 2, wherein the composition optionally comprises a pH adjuster.

7. The composition according to claim 1 or 2, wherein the composition is a suspension; the suspension comprises 2-10 mg/mL, preferably 2-8 mg/mL, of the docetaxel; the suspension has a pH value of 3.4-5.8, preferably 3.6-5.6, or 3.8-5.0, and more preferably 3.9-4.8; the suspension comprises 0-1.8% (w/v), preferably 0.45%-1.8% (w/v), and more preferably 0.9%-1.8% (w/v), of sodium chloride.

8. A lyophilized powder, prepared from the composition according to claim 7.

9. The composition according to claim 1 or 2, wherein the composition is a lyophilized powder; the lyophilized powder comprises sodium chloride, and the sodium chloride and the docetaxel are in a weight ratio of (0.75-9):1, preferably (1-7):1, preferably (1.5-4.5):1, and most preferably 2.25:1; the lyophilized powder is obtained by lyophilizing a suspension comprising the docetaxel albumin nanoparticle; the suspension has a pH of 3.4-5.8, preferably 3.6-5.6, or 3.8-5.0, and more preferably 3.9-4.8.

10. A reconstituted suspension, obtained by reconstituting the lyophilized powder according to claim 8 or the composition according to claim 9 with a reconstitution medium; the reconstitution medium is selected from water for injection, a sodium chloride solution, and a glucose solution, preferably water for injection; the reconstituted suspension has a pH of 3.4-5.8, preferably 3.6-5.6, or 3.8-5.0, and more preferably 3.9-4.8.

11. A drug, prepared from the composition according to any one of claims 1-7 and 9, or the lyophilized powder according to claim 8, or the reconstituted suspension according to claim 10, wherein the drug is in a clinically acceptable dosage form, preferably an injection, and more preferably a liquid injection or a lyophilized powder injection.

12. A method for preparing a composition comprising a docetaxel albumin nanoparticle, comprising the following steps:
(1) dissolving docetaxel in an organic solvent to obtain an organic phase solution;
(2) taking a human serum albumin solution, and adding an acid to adjust the pH value to obtain an aqueous phase solution of an acid-denatured albumin; taking another salt solution;
(3) mixing the organic phase solution, the aqueous phase solution, and the salt solution for loading the drug to obtain a drug-loading solution; and
(4) dialyzing;
wherein
step (2) optionally comprises a step of diluting the human serum albumin solution with water for injection to obtain an albumin dilution before adding the acid to adjust the pH value;
step (2) optionally comprises a step of incubating after adding the acid to adjust the pH value;
step (3) optionally comprises a step of cooling after mixing for loading the drug;
step (4) optionally comprises a step of concentrating the drug-loading solution obtained in step (3) to obtain a concentrated solution before dialyzing;
step (4) optionally comprises a step of concentrating or diluting after dialyzing;
after step (4), the method optionally comprises step (5) of sterilizing and filtering; and after step (5), the method optionally comprises step (6) of lyophilizing.

13. The preparation method according to claim 12, wherein in step (1), the docetaxel is in any form, preferably anhydrous docetaxel, docetaxel hemihydrate, or docetaxel trihydrate; based on the anhydrous docetaxel, the docetaxel and the human serum albumin are in a mass ratio of 1:(2.0-10.0), preferably 1:(3.0-7.0), and more preferably 1:(4.0-6.0);
alternatively, in step (1), the organic solvent is selected from a water-miscible solvent, preferably ethanol, methanol, acetone, and dimethyl sulfoxide, and more preferably ethanol; the organic phase solution comprises 45-90 mg/mL(on an anhydrous basis), preferably 45-70 mg/mL of the docetaxel (on an anhydrous basis).

14. The preparation method according to claim 12, wherein in step (2), the content of sodium caprylate in the human serum albumin solution is no more than 0.12 mmol/g protein, preferably no more than 0.08 mmol/g protein, and preferably 0.045-0.08 mmol/g protein;
alternatively, in step (2), the acid is selected from an acidic amino acid or acidic polypeptide, an organic acid, and an inorganic acid; the acidic amino acid or acidic polypeptide comprises cysteine hydrochloride and glutathione; the organic acid comprises citric acid and tartaric acid; the inorganic acid comprises hydrochloric acid and sulfuric acid; the acid is preferably cysteine hydrochloride, glutathione, or hydrochloric acid, and more preferably cysteine hydrochloride; the pH value is preferably 3.5-5.5, preferably 3.5-5.0, more preferably 3.8-4.7, and more preferably 4.0-4.5;
alternatively, in step (2), the incubating refers to heating to 35-42 °C, preferably 38-42 °C after adding the acid to adjust the pH value, and incubating for more than 30 min, preferably 30-60 min.
alternatively, in step (2), a salt of the salt solution is selected from sodium chloride, potassium chloride, sodium sulfate, and magnesium sulfate, preferably sodium chloride; the salt solution is at a concentration of no less than 2%, preferably 2%-35%, and more preferably 10%-20%;
alternatively, in step (2), the albumin dilution is a solution comprising 6-25 mg/mL of albumin, preferably a solution comprising 10-20 mg/mL of albumin, more preferably a solution comprising 12-18 mg/mL of albumin, and more preferably a solution comprising 15 mg/mL of albumin.

15. The preparation method according to claim 12, wherein in step (3), the drug loading is performed under a condition that the organic phase solution and the aqueous phase solution are heated to 35-42 °C, preferably 38-42 °C, and the organic phase solution, the aqueous phase solution, and the salt solution are mixed for loading the drug;
alternatively, in step 3, the cooling refers to cooling to room temperature, preferably 0-20 °C, and more preferably 7-15 °C.

16. The preparation method according to claim 12, wherein in step (4), the dialyzing is performed using a sodium chloride solution as a dialysate; the sodium chloride solution is at a concentration of no more than 1.8% (w/v), preferably 0.45%-1.8% (w/v), and more preferably 0.9%-1.8% (w/v);
alternatively, the dialyzing in step (4) is performed using a dialysis membrane with a molecular weight cutoff of 10-50 kDa, preferably 10-30 kDa, and more preferably 10 kDa or 30 kDa;
alternatively, in step (4), the dialysate used in the dialysis has a volume of no less than 3 times, preferably 3-10 times, and more preferably 3-6 times that of the drug-loading solution or the concentrated solution;
alternatively, in step (4), the concentrated solution comprises 4-10 mg/mL, preferably 6-10 mg/mL, more preferably 7-9 mg/mL, and more preferably 8 mg/mL, of the docetaxel.

17. The preparation method according to claim 12, wherein the human serum albumin solution in step (2) is prepared by the following method: diluting a commercially available human serum albumin solution with water for injection or normal saline to obtain a diluted albumin solution; dialyzing the diluted albumin solution with water for injection or normal saline as a dialysate, and partially removing sodium caprylate to obtain a human serum albumin solution with low sodium caprylate content; the human serum albumin solution with low sodium caprylate content is directly used as the human serum albumin solution in step (2), or mixed with a human serum albumin solution with an additional sodium caprylate content in proportion to obtain a desired content, and then used as the human serum albumin solution in step (2).

18. The preparation method according to claim 17, wherein the human serum albumin solution is diluted with a dilution factor of no less than 4, preferably 4-7;
alternatively, the dialyzing is performed using a dialysis membrane with a molecular weight cutoff of 10-50 kDa, preferably 10-30 kDa, and more preferably 10 kDa or 30 kDa; the dialysate has a volume of more than 3 times, preferably 3-10 times, and more preferably 3-6 times that of the diluted albumin solution;
alternatively, the human serum albumin solution with low sodium caprylate content comprises less than 0.16 mmol/g protein, preferably less than 0.12 mmol/g protein, preferably less than 0.10 mmol/g protein, and preferably less than 0.08 mmol/g protein, of sodium caprylate.

19. A composition comprising a docetaxel albumin nanoparticle, prepared by the method according to any one of claims 12-18.

20. A drug, prepared from the composition comprising the docetaxel albumin nanoparticle according to claim 19, wherein the drug is in a clinically acceptable dosage form, preferably an injection, and further preferably a liquid injection or a lyophilized powder injection.

21. A method for reducing the content of sodium caprylate in a human serum albumin solution, wherein the method comprises diluting a commercially available human serum albumin solution with water for injection or normal saline to obtain a diluted albumin solution; and dialyzing the diluted albumin solution with water for injection or normal saline as a dialysate;
preferably, the human serum albumin solution is diluted with a dilution factor of no less than 4, preferably 4-7;
preferably, the dialyzing is performed using a dialysis membrane with a molecular weight cutoff of 10-50 kDa, preferably 10-30 kDa, and more preferably 10 kDa or 30 kDa; the dialysate has a volume of more than 3 times, preferably 3-10 times, and more preferably 3-6 times that of the diluted albumin solution.
